# EUROPEAN PATENT APPLICATION

(11) **EP 4 578 405 A1**
(43) Date of publication of application: **02.07.2025**
(21) Application number: 24223449.0
(22) Date of filing: 27.12.2024
(51) Int. Cl.: A61B 8/08, A61B 8/00, G06T 7/00

(54) **ULTRASONIC DIAGNOSTIC APPARATUS**

(30) Priority: 26.12.2023 JP 2023219603; 25.11.2024 JP 2024204640
(71) Applicant: Canon Medical Systems Corporation, Tochigi 324-0036 (JP)
(72) Inventor: AMAGI, Seina, Otawara-shi, 324-0036 (JP); TAKADA, Yuko, Otawara-shi, 324-0036 (JP); WATANABE, Masaki, Otawara-shi, 324-0036 (JP)
(74) Representative: Marks & Clerk LLP

(57) **Abstract**

An ultrasonic diagnostic apparatus according to an embodiment includes a processing circuitry. The processing circuitry is configured to acquire ultrasound images of a plurality of frames by continuous scanning of a subject using an ultrasonic probe. The processing circuitry is further configured to distinguishably display positions on a time axis, where ultrasound image groups have been acquired, for each ultrasound image group which includes two or more frames which include a common examination object among the plurality of frames.

## Description

### CROSS REFERENCE TO RELATED APPLICATIONS

This application is based upon and claims the benefit of priority from the prior Japanese Patent Applications No. 2023-219603, filed on December 26, 2023, and No. 2024-204640, filed on November 25, 2024.

### FIELD

Embodiments disclosed in the present specification and drawings relate to an ultrasonic diagnostic apparatus.

### BACKGROUND

Thus far, an ultrasonic diagnostic apparatus has been used to observe growth of a fetus. For example, based on ultrasound images, the ultrasonic diagnostic apparatus detects examination objects such as a biparietal diameter (BPD), an abdominal circumference (AC), and a femur length (FL) of a fetus. Then, based on the result of detection of the examination object, the ultrasonic diagnostic apparatus performs estimation of the weight of the fetus, etc.

However, it has been the case that a conventional ultrasonic diagnostic apparatus performs visualization of ultrasound images, freezing, and detection of the examination object individually for examination objects. Thus, the conventional ultrasonic diagnostic apparatus needs to repeat the operation of an ultrasonic probe on the mother's body and the operation of an apparatus main body individually for examination objects. Therefore, it has been difficult for the conventional ultrasonic diagnostic apparatus to simply and quickly perform examination of examination objects based on ultrasound images.

### SUMMARY OF INVENTION

An ultrasonic diagnostic apparatus including a processing circuitry configured to:
acquire ultrasound images of a plurality of frames by continuous scanning of a subject using an ultrasonic probe; and
distinguishably display positions on a time axis, where ultrasound image groups have been acquired, for each ultrasound image group which includes two or more frames which include a common examination object among the plurality of frames.

The processing circuitry may further configured to:
distinguish the examination object for each of the plurality of frames; and
distinguishably display the positions on the time axis where the ultrasound image groups have been acquired, for each ultrasound image group, based on a result of distinguishing the examination object.

The processing circuitry may be further configured to: calculate, for each frame in which the examination object has been distinguished, a degree of appropriateness indicating how appropriate the examination is; and
determine, based on the calculated degrees of appropriateness, an ultrasound image for performing examination of the examination object.

The processing circuitry may be further configured to: distinguish the examination object by dividing the ultrasound images of the plurality of frames into blocks each showing a common attribute;
calculate the degree of appropriateness for each of the divided blocks; and
determine, for each of the divided blocks, based on the calculated degrees of appropriateness, an ultrasound image for performing examination of the examination object.

The common attribute may be a common examination object, and
the processing circuitry may be further configured to: calculate a degree of certainty that a cross section corresponding to the examination object will be included in the ultrasound image; and
divide, based on the calculated degrees of certainty, the ultrasound images of the plurality of frames into the blocks each showing the common examination object.

The common attribute may be a common time phase, and
the processing circuitry may be further configured to divide, based on time, the ultrasound images of the plurality of frames into the blocks each showing a common time phase.

The processing circuitry may be further configured to distinguishably display the positions on the time axis by displaying a block image showing the divided block.

The processing circuitry may be further configured to vary a display color of the block image according to the calculated degree of certainty.

The processing circuitry may be further configured to display the block image in gradation according to the calculated degree of certainty.

The processing circuitry may be further configured to replace, as an ultrasound image for performing examination of the examination object, an ultrasound image of a frame selected by a user from the displayed block image with a determined ultrasound image for performing examination of the examination object.

The processing circuitry may be further configured to: divide the ultrasound images of the plurality of frames into the blocks in real time during the scanning; and
display the block image together with the ultrasound image in real time during the scanning.

The processing circuitry may be further configured to, in a case where a plurality of examination objects are simultaneously included in the ultrasound images of the plurality of frames, divide the ultrasound images of the plurality of frames into the blocks individually for the plurality of examination objects, and
display the block images of the plurality of examination objects in an overlapping manner or in a side-by-side manner.

The processing circuitry may be further configured to display a graph showing the calculated degree of certainty.

The processing circuitry may be further configured to: contract the block based on detection values of the examination object included in the block; and
calculate the degree of appropriateness for the contracted block.

The processing circuitry may be further configured to calculate the degree of appropriateness based on a detection value of the examination object.

The block image may be an image schematically showing the block, and
the processing circuitry may be further configured to arrange and display the plurality of block images according to a time series, and display, on each block image, a frame of which the calculated degree of certainty is largest.

The processing circuitry may be further configured to vary a display color of the block image according to the examination object.

The block image may be a thumbnail of the ultrasound image of each of the blocks, and
the processing circuitry may be further configured to display the thumbnail such that the thumbnail is selectable by an operation of a user, and display the ultrasound image corresponding to the thumbnail selected by an operation of the user.

The block image may be an image showing a head frame number and a tail frame number in the block.

The processing circuitry may be further configured to display, according to an operation of a user, a determined ultrasound image for performing examination of the examination object.

The subject may be a mother's body, and
the examination object may be a dimension of a fetus in the mother's body.

### BRIEF DESCRIPTION OF THE DRAWINGS

FIG. 1 is a block diagram showing a configuration example of an ultrasonic diagnostic apparatus according to a first embodiment;
FIG. 2 is a flowchart showing an operation example of the ultrasonic diagnostic apparatus according to the first embodiment;
FIG. 3 is a diagram showing a step of generating ultrasound images in the operation example of the ultrasonic diagnostic apparatus according to the first embodiment;
FIG. 4 is a diagram showing a step of displaying ultrasound images in the operation example of the ultrasonic diagnostic apparatus according to the first embodiment;
FIG. 5 is a diagram showing a step of calculating degrees of certainty in the operation example of the ultrasonic diagnostic apparatus according to the first embodiment;
FIG. 6 is a diagram showing a step of performing blocking on ultrasound images in the operation example of the ultrasonic diagnostic apparatus according to the first embodiment;
FIG. 7 is a diagram showing a step of displaying block images in the operation example of the ultrasonic diagnostic apparatus according to the first embodiment;
FIG. 8 is a diagram showing a step of calculating degrees of appropriateness in the operation example of the ultrasonic diagnostic apparatus according to the first embodiment;
FIG. 9 is a diagram showing a step of displaying block images in an operation example of an ultrasonic diagnostic apparatus according to a first modification example of the first embodiment;
FIG. 10 is a diagram showing a step of displaying block images in an operation example of an ultrasonic diagnostic apparatus according to a second modification example of the first embodiment;
FIG. 11 is a flowchart showing an operation example of an ultrasonic diagnostic apparatus according to a third modification example of the first embodiment;
FIG. 12 is a diagram showing a step of displaying thumbnails in the operation example of the ultrasonic diagnostic apparatus according to the third modification example of the first embodiment;
FIG. 13 is a diagram showing check boxes of detected examination objects in an operation example of an ultrasonic diagnostic apparatus according to a fourth modification example of the first embodiment;
FIG. 14 is a diagram showing a step of displaying block images in an operation example of an ultrasonic diagnostic apparatus according to a fifth modification example of the first embodiment;
FIG. 15 is a diagram showing a step of displaying a graph showing degrees of certainty in an operation example of an ultrasonic diagnostic apparatus according to a sixth modification example of the first embodiment;
FIG. 16 is a diagram showing a step of displaying block images in an operation example of an ultrasonic diagnostic apparatus according to a seventh modification example of the first embodiment;
FIG. 17 is a diagram showing a step of displaying thumbnails in an operation example of an ultrasonic diagnostic apparatus according to an eighth modification example of the first embodiment;
FIG. 18 is a flowchart showing an operation example of an ultrasonic diagnostic apparatus according to a ninth modification example of the first embodiment;
FIG. 19 is a diagram showing a step of contracting a block in the operation example of the ultrasonic diagnostic apparatus according to the ninth modification example of the first embodiment;
FIG. 20 is a block diagram showing a configuration example of an ultrasonic diagnostic apparatus according to a second embodiment;
FIG. 21 is a flowchart showing an operation example of the ultrasonic diagnostic apparatus according to the second embodiment;
FIG. 22 is a flowchart showing an operation example of an ultrasonic diagnostic apparatus according to a third embodiment;
FIG. 23 is a diagram showing a blocking step in the operation example of the ultrasonic diagnostic apparatus according to the third embodiment; and
FIG. 24 is a diagram showing a step of displaying ultrasound images for performing examination of examination objects in the operation example of the ultrasonic diagnostic apparatus according to the third embodiment.

### DETAILED DESCRIPTION

Hereinbelow, embodiments of an ultrasonic diagnostic apparatus are described with reference to the drawings. Note that in the following description, constituent elements having substantially the same functions and configurations are denoted by the same reference numerals, and a repeated description is made only when necessary.

### (First embodiment)

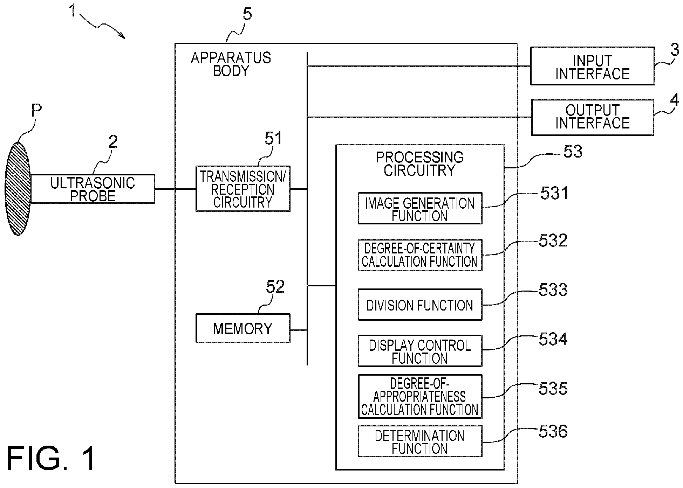

FIG. 1 is a block diagram showing an example of a configuration of an ultrasonic diagnostic apparatus 1 according to a first embodiment. As shown in FIG. 1, the ultrasonic diagnostic apparatus 1 according to the first embodiment includes an ultrasonic probe 2, an input interface 3, an output interface 4, and an apparatus body 5. The ultrasonic probe 2, the input interface 3, and the output interface 4 are connected to be able to communicate with the apparatus body 5.

The ultrasonic probe 2 is a device that, in order to acquire ultrasound images of a subject P, transmits ultrasonic waves to the subject P and receives reflected waves (echoes) of the ultrasonic waves from the subject P. The subject P is, for example, a mother's body.

The ultrasonic probe 2 includes a plurality of transducers. The plurality of transducers generate ultrasonic waves based on a drive signal such as a voltage supplied from the apparatus body 5. The ultrasonic probe 2 receives reflected waves from the subject P, and converts the reflected waves into an electric signal. That is, the ultrasonic probe 2 scans the subject P with ultrasonic waves, and receives reflected waves from the subject P. The transducer is provided with electrodes for supplying a drive signal and inputting an electric signal of reflected waves. The transducer may be formed of, for example, PZT (lead zirconate titanate) and PVDF (polyvinylidene fluoride), or the like. An acoustic matching layer and an acoustic lens, for example, are placed on the front surface of the transducer. A backing material, for example, is placed on the back surface of the transducer. The acoustic matching layer is called also a λ/4 layer, and is a layer for transmitting and receiving ultrasonic waves with good efficiency by reducing the impedance difference between the transducer and the living body. The acoustic lens is a structure for reducing friction with the living body surface during examination and for focusing an ultrasonic beam to improve slice resolution. The backing material is a structure that absorbs rearward ultrasonic waves and shortens the pulse width of forward ultrasonic waves. The ultrasonic probe 2 is connected to the apparatus body 5 in an attachable and detachable manner.

When ultrasonic waves are transmitted from the ultrasonic probe 2 to the subject P, the transmitted ultrasonic waves are reflected one after another at surfaces of internal body tissue of the subject P at which acoustic impedance is discontinuous, and are received as a reflected wave signal by the plurality of transducers included in the ultrasonic probe 2. The amplitude of the received reflected wave signal depends on the difference in acoustic impedance at the discontinuous surface at which ultrasonic waves are reflected. Note that a reflected wave signal in the case where transmitted ultrasonic pulses are reflected by the moving blood flow or the surface of the heart wall or the like receives a frequency shift depending on the velocity component with respect to the direction of ultrasonic transmission of the moving body, due to the Doppler effect.

The ultrasonic probe 2 is, for example, a three-dimensional probe that three-dimensionally scans the subject P, that is, a mechanical 4D probe or a 2D array probe. The ultrasonic probe 2 may be a 1D array probe that two-dimensionally scans the subject P.

The input interface 3 accepts operations of inputting various instructions and various pieces of information from the operator (that is, the user). Specifically, the input interface 3 converts an input operation accepted from the operator into an electric signal, and outputs the electric signal to the apparatus body 5. For example, the input interface 3 is obtained by using a trackball, a switch button, a mouse, a keyboard, a touch pad that performs an input operation by touching on an operating surface, a touch screen in which a display screen and a touch pad are integrated, a non-contact input circuitry using an optical sensor, a sound input circuitry, etc. Note that the input interface 3 is not limited to one including physical operating components such as a mouse or a keyboard. For example, also an electric signal processing circuitry that receives an electric signal corresponding to an input operation from an external input device provided separately from the apparatus and outputs the electric signal to the control circuitry is included in examples of the input interface 3.

The output interface 4 outputs various pieces of information. For example, the output interface 4 includes a display. The display converts data of information and images sent from the apparatus body 5 into an electric signal for displaying, and outputs the electric signal. The display is obtained by using a liquid crystal monitor, a CRT (cathode ray tube) monitor, a touch panel, or the like. The output interface 4 may include a speaker. The speaker outputs a predetermined sound such as a beep sound in order to notify the operator of the processing status of the apparatus body 5.

The apparatus body 5 includes a transmission/reception circuitry 51, a memory 52, and a processing circuitry 53.

The transmission/reception circuitry 51 is a circuitry that supplies a drive signal to the ultrasonic probe 2 under the control of the processing circuitry 53. The transmission/reception circuitry 51 is also a circuitry that performs various pieces of processing on a reflected wave signal received by the ultrasonic probe 2 and generates reflected wave data.

In order to supply a drive signal to the ultrasonic probe 2, the transmission/reception circuitry 51 includes, for example, a pulse generator, a transmission delay part, a pulser, etc. The pulse generator repeatedly generates rate pulses for forming transmission ultrasonic waves at a predetermined rate frequency. The transmission delay part focuses ultrasonic waves generated from the ultrasonic probe 2 into a beam shape, and gives, to each rate pulse generated by the pulse generator, a delay time for each transducer necessary to determine transmission directivity. The pulser applies a drive signal (drive pulse) to the ultrasonic probe 2 at timing based on the rate pulse to which a delay time is given. That is, the transmission delay part changes the delay time to be given to each rate pulse, and thereby arbitrarily adjusts the transmission direction of ultrasonic waves transmitted from the transducer surface.

Further, in order to perform various pieces of processing on a reflected wave signal received by the ultrasonic probe 2 and generate reflected wave data, the transmission/reception circuitry 51 includes, for example, a preamplifier, an A/D (analog/digital) converter, a reception delay part, an adder, etc. The preamplifier amplifies a reflected wave signal for each channel. The A/D converter performs A/D conversion on the amplified reflected wave signal. The reception delay part gives a delay time necessary to determine reception directivity. The adder performs processing of adding reflected wave signals processed by the reception delay part, and generates reflected wave data. By the addition processing of the adder, a reflection component from a direction according to the reception directivity of the reflected wave signal is emphasized, and a comprehensive beam of ultrasonic transmission and reception is formed by the reception directivity and the transmission directivity. As the form of the output signal from the transmission/reception circuitry 51, various forms can be selected, such as a case where the output signal is a signal including phase information called an RF (radio frequency) signal and a case where the output signal is amplitude information after envelope detection processing.

In the example shown in FIG. 1, the transmission/reception circuitry 51 is placed in the apparatus body 5. The transmission/reception circuitry 51 is not limited to being placed in the apparatus body 5, and at least part of the transmission/reception circuitry 51 may be placed in the ultrasonic probe 2.

The memory 52 is a non-transitory storage device that stores various pieces of information, and is, for example, an HDD (hard disk drive), an optical disk, an SSD (solid state drive), an integrated circuit storage device, or the like. The memory 52 stores, for example, a control program for controlling the ultrasonic diagnostic apparatus 1 and various pieces of data used to execute the control program. In addition to the HDD, the SSD, and the like, the memory 52 may be a drive device that performs reading and writing of various pieces of information with a portable storage medium such as a CD (compact disc), a DVD (digital versatile disc), or a flash memory, a semiconductor memory element such as a RAM (random-access memory), or the like.

The processing circuitry 53 is a circuitry that controls the entire operation of the ultrasonic diagnostic apparatus 1 according to an electric signal of an input operation inputted from the input interface 3. For example, the processing circuitry 53 includes an image generation function 531 that is an example of an acquisition part, a degree-of-certainty calculation function 532, a division function 533, a display control function 534, a degree-of-appropriateness calculation function 535, and a determination function 536.

Here, for example, each of the processing functions to be executed by the image generation function 531, the degree-of-certainty calculation function 532, the division function 533, the display control function 534, the degree-of-appropriateness calculation function 535, and the determination function 536, which are constituent elements of the processing circuitry 53 shown in FIG. 1, is recorded in the memory 52 in a form of a program executable by a computer. The processing circuitry 53 is, for example, a processor. The processor forming the processing circuitry 53 reads each program from the memory 52, and executes the program to implement a function corresponding to the read program. In other words, the processing circuitry 53 in a state where it has read each program has the corresponding function shown in the processing circuitry 53 of FIG. 1. The processing circuitry 53 may include a circuitry other than the processor.

Note that, although FIG. 1 shows a case where the processing functions of the image generation function 531, the degree-of-certainty calculation function 532, the division function 533, the display control function 534, the degree-of-appropriateness calculation function 535, and the determination function 536 are implemented by a single processing circuitry 53, the embodiment is not limited thereto. For example, the processing circuitry 53 may be configured by combining a plurality of independent processors, and each processor may execute a corresponding program to implement a corresponding processing function. Further, the processing functions included in the processing circuitry 53 may be implemented by being distributed or integrated in a single processing circuitry or a plurality of processing circuitries as appropriate.

The image generation function 531 generates ultrasound images according to scanning of the subject P using the ultrasonic probe 2. More specifically, the image generation function 531 sequentially generates (that is, acquires) ultrasound images of a plurality of frames according to continuous scanning of the subject P using the ultrasonic probe 2. The continuous scanning is, for example, scanning in which a freeze operation of stopping a moving ultrasound image in the middle is not performed.

The image generation function 531 receives reflected wave data from the transmission/reception circuitry 51, performs logarithmic amplification, envelope detection processing, etc., and generates data (B-mode data) in which signal intensity is expressed by brightness of luminance, for example. Further, the image generation function 531 performs frequency analysis on velocity information from reflected wave data received from the transmission/reception circuitry 51, extracts blood flow, tissue, and contrast medium echo components based on the Doppler effect, and generates data (Doppler data) in which moving body information such as velocity, variance, and power is extracted for multiple points. The image generation function 531 may be one capable of processing both two-dimensional reflected wave data and three-dimensional reflected wave data. That is, the image generation function 531 may generate two-dimensional B-mode data from two-dimensional reflected wave data and generate three-dimensional B-mode data from three-dimensional reflected wave data. Further, the image generation function 531 may generate two-dimensional Doppler data from two-dimensional reflected wave data and generate three-dimensional Doppler data from three-dimensional reflected wave data.

Then, the image generation function 531 generates, from B-mode data, a B-mode image in which the intensity of reflected waves is shown by luminance. Further, for example, the image generation function 531 generates, from Doppler data, a Doppler image in which blood flow information is imaged. The Doppler image is velocity image data showing the average velocity of blood flow, variance image data showing the variance value of blood flow, power image data showing the power of blood flow, or image data in which these are combined. Further, as a Doppler image, the image generation function 531 generates a color Doppler image in which blood flow information such as the average velocity, variance value, or power of blood flow is displayed in color, or generates a Doppler image in which one piece of blood flow information is displayed in a gray scale. Further, for example, the image generation function 531 can generate an M-mode image from time-series data of B-mode data on one scanning line. Further, the image generation function 531 can generate, from Doppler data, a Doppler waveform in which velocity information of blood flow or tissue is plotted along a time series.

The degree-of-certainty calculation function 532 calculates a degree of certainty that a cross section corresponding to the examination object will be included in the ultrasound image generated by the image generation function 531. The examination object can be referred to also as a measurement item. The degree of certainty can be referred to also as a probability. The examination object is, for example, BPD, AC, FL, or the like of a fetus in a mother's body. That is, the examination object is a dimension of a fetus. The examination object may further include a head circumference (HC), a humerus length (HL), or the like. The degree-of-certainty calculation function 532 calculates the degree of certainty for each frame, for example. Alternatively, the degree-of-certainty calculation function 532 may calculate the degree of certainty for each predetermined number of frames.

From the ultrasound image, the degree-of-certainty calculation function 532 detects, for example, the types and positions of a part corresponding to the examination object and a structure included in the part. The degree-of-certainty calculation function 532 may detect the types and positions of such a part and such a structure by training the part and the structure using machine learning. The degree-of-certainty calculation function 532 causes the memory 52 to store the detected types and positions of such a part and such a structure. Based on feature values such as the types and positions of such a part and such a structure stored in the memory 52, the degree-of-certainty calculation function 532 calculates a degree of certainty that a cross section corresponding to the examination object will be included in the ultrasound image. The degree-of-certainty calculation function 532 may calculate the degree of certainty of the ultrasound image of each frame by training ultrasound images including cross sections corresponding to the examination object using machine learning.

The division function 533 divides ultrasound images of a plurality of frames into blocks each showing a common attribute. Here, the division means classifying ultrasound images of a plurality of frames into a plurality of blocks based on common attributes among the ultrasound images. Ultrasound images having no common attributes are not classified into any block. In the first embodiment, the common attribute is a common examination object (for example, BPD, AC, FL, or the like). That is, in the first embodiment, based on the degrees of certainty calculated by the degree-of-certainty calculation function 532, the division function 533 divides ultrasound images of a plurality of frames into blocks each showing a common examination object. In other words, the division function 533 distinguishes the examination object for each of the plurality of frames based on the common attribute.

The display control function 534 displays, on a display, block images showing blocks divided by the division function 533. That is, the display control function 534 distinguishably displays positions on a time axis, where ultrasound image groups have been acquired, for each ultrasound image group which is composed of two or more frames which include a common examination object among the plurality of frames, based on a result of distinguishing the examination object. With this configuration, it is easy to determine ultrasound images for performing examination for each examination object (i.e., each ultrasound image group), which makes examination simpler and quicker. The display control function 534 further displays, on the display, ultrasound images generated by the image generation function 531. The block image may be an image schematically showing a block. The display control function 534 may arrange and display a plurality of block images according to a time series. The display control function 534 may vary the display color of the block image according to the examination object.

The degree-of-appropriateness calculation function 535 calculates, for each of ultrasound images of a plurality of frames acquired by continuous scanning of the subject P using the ultrasonic probe 2, a degree of appropriateness indicating how appropriate the examination for the examination object is. In the first embodiment, the degree-of-appropriateness calculation function 535 calculates the degree of appropriateness for each block (that is, for each examination object) divided by the division function 533. The degree-of-appropriateness calculation function 535 calculates the degree of appropriateness for each frame, for example. Alternatively, the degree-of-appropriateness calculation function 535 may calculate the degree of appropriateness for each predetermined number of frames (for example, at intervals of five frames or the like). The degree-of-appropriateness calculation function 535 may calculate a degree of appropriateness proportional to the degree of certainty calculated by the degree-of-certainty calculation function 532. Alternatively, the degree-of-appropriateness calculation function 535 may calculate a degree of appropriateness proportional to the difference (that is, the deviation) between the degree of certainty of a frame for which the degree of appropriateness is to be calculated and the average value of the degrees of certainty in the block that the frame belongs to. Alternatively, the degree-of-appropriateness calculation function 535 may automatically detect (that is, automatically measure) an examination object (that is, a measurement item), and may calculate the degree of appropriateness based on the result of automatic detection. In this case, the degree-of-appropriateness calculation function 535 may calculate a degree of appropriateness proportional to the detection value.

The determination function 536 determines, based on the degrees of appropriateness calculated by the degree-of-appropriateness calculation function 535, ultrasound images for performing examination of examination objects from among the ultrasound images of a plurality of frames. For example, the determination function 536 determines that the ultrasound image of a frame having the largest degree of appropriateness in a block is an ultrasound image for performing examination of the examination object corresponding to the block. The frame having the largest degree of appropriateness in the block may be a frame having a degree of appropriateness exceeding a preset threshold. When ultrasound images of two or more frames in the block have degrees of appropriateness exceeding a threshold, the determination function 536 may determine that they are ultrasound images for performing examination of the examination object, and may recommend them to the user. In this case, the user may select an arbitrary ultrasound image from the recommended ultrasound images of two or more frames, and may use the selected ultrasound image for examination of the examination object.

The examination of the examination object is, for example, examination of BPD, AC, or FL of a fetus in a mother's body. The examination of the examination object can be replaced with, out of automatic detection (that is, automatic measurement) of the examination object already performed by the degree-of-appropriateness calculation function 535, automatic detection of the examination object performed on the ultrasound image of a frame having the largest degree of appropriateness. In this case, the result of automatic detection of the examination object by the degree-of-appropriateness calculation function 535 is equivalent to a result of examination of the examination object, and thus it is not necessary to repeat examination of the examination object. In this case, the "determination of an ultrasound image for performing examination of the examination object" performed by the determination function 536 is synonymous with "determination of the ultrasound image of a frame that has undergone automatic detection of the examination object and that has the largest degree of appropriateness". Alternatively, the examination of the examination object may be examination performed separately from automatic detection of the examination object by the degree-of-appropriateness calculation function 535. In this case, the examination of the examination object may be, for example, examination having higher accuracy than automatic detection of the examination object or examination using a different method for calculating the examination object. The processing circuitry 53 may further include a function of performing examination of the examination object and a function of estimating the weight of the fetus using a result of examination of the examination object.

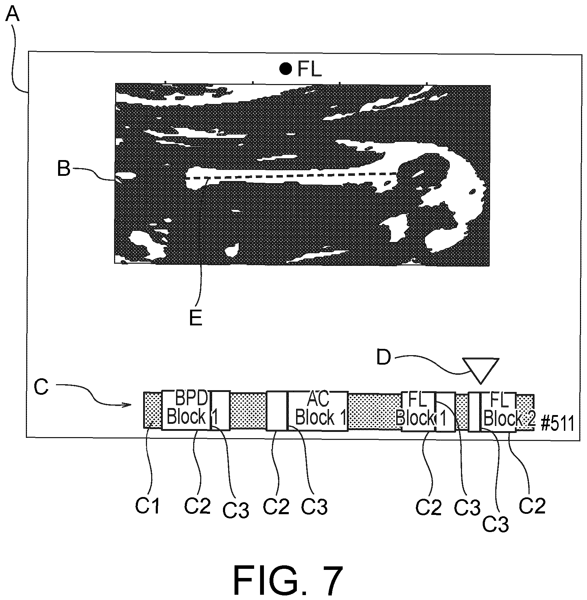

In the case where the processing circuitry 53 includes a function of performing examination of the examination object (hereinafter, also referred to as an examination function), the examination function may automatically execute examination on the ultrasound image determined by the determination function 536. Alternatively, the examination function may execute examination on an examination object in a block image C2 designated by the user on a belt-shaped image C1 (hereinafter, also referred to as a timeline) representing a time axis, which is shown in FIG. 7, etc. described later.

The display control function 534, according to an operation of the operator, displays the ultrasound image for performing examination of the examination object determined by the determination function 536. In the case where the examination function automatically executes examination, the display control function 534 may display the examination result on the display according to an operation by the user. Further, the display control function 534 may display, on the display, an ultrasound image designated by the user on the timeline C1. That is, the ultrasound image displayed on the display may jump from an ultrasound image currently displayed according to a predetermined displaying order or designation by the user to an ultrasound image designated by the user on the timeline C1.

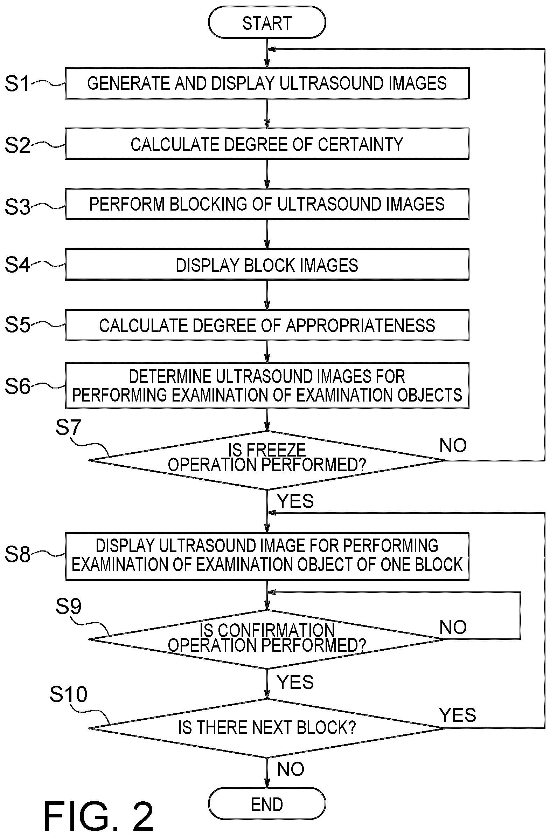

Next, an operation example of the ultrasonic diagnostic apparatus 1 according to the first embodiment configured as above is described. FIG. 2 is a flowchart showing an operation example of the ultrasonic diagnostic apparatus 1 according to the first embodiment. Note that the series of steps shown in the flowchart of FIG. 2 is repeated as necessary.

First, as shown in FIG. 2, according to an operator continuously scanning a subject by using the ultrasonic probe 2, the image generation function 531 sequentially generates ultrasound images of frames (step S1). The display control function 534 sequentially displays, on the display, ultrasound images of frames generated by the image generation function 531, in a moving image form (step S1).

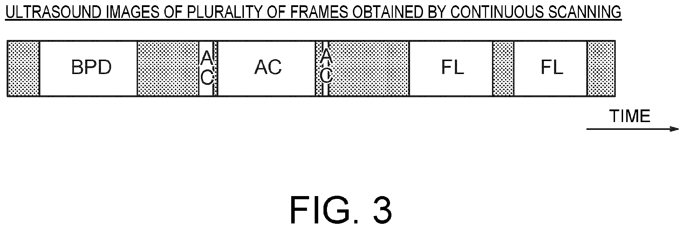

FIG. 3 is a diagram showing a step of generating ultrasound images in the operation example of the ultrasonic diagnostic apparatus 1 according to the first embodiment. In the example shown in FIG. 3, the image generation function 531 generates ultrasound images of a plurality of frames according to continuous scanning (that is, one scan). In the example shown in FIG. 3, the ultrasound images of a plurality of frames include ultrasound images of a series of frames corresponding to BPD, ultrasound images of a series of frames corresponding to AC, and ultrasound images of a series of frames corresponding to FL.

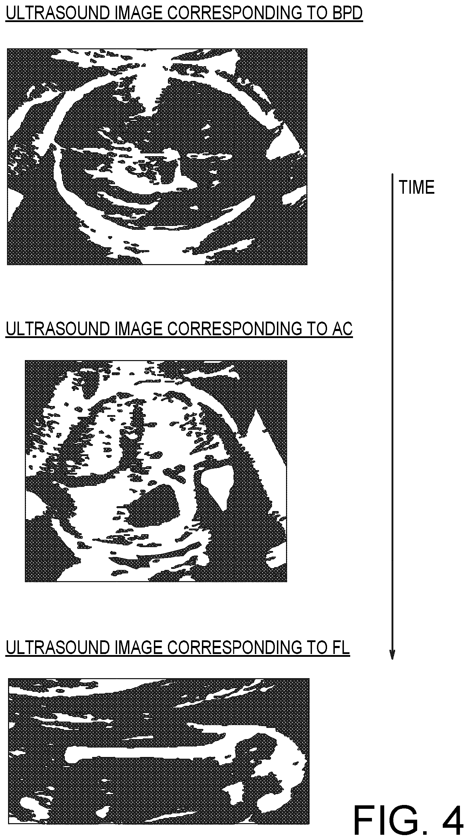

FIG. 4 is a diagram showing a step of displaying ultrasound images in the operation example of the ultrasonic diagnostic apparatus 1 according to the first embodiment. In the example shown in FIG. 4, according to the lapse of time, the display control function 534 sequentially displays ultrasound images corresponding to BPD, ultrasound images corresponding to AC, and ultrasound images corresponding to FL. The ultrasound image corresponding to BPD includes a cross section corresponding to BPD, that is, a measurement cross section with which BPD can be measured. The ultrasound image corresponding to AC includes a cross section corresponding to AC, that is, a measurement cross section with which AC can be measured. The ultrasound image corresponding to FL includes a cross section corresponding to FL, that is, a measurement cross section with which FL can be measured.

After ultrasound images of a predetermined number of frames in series are generated and displayed, as shown in FIG. 2, the degree-of-certainty calculation function 532 calculates the degree of certainty of the ultrasound image of each frame (step S2).

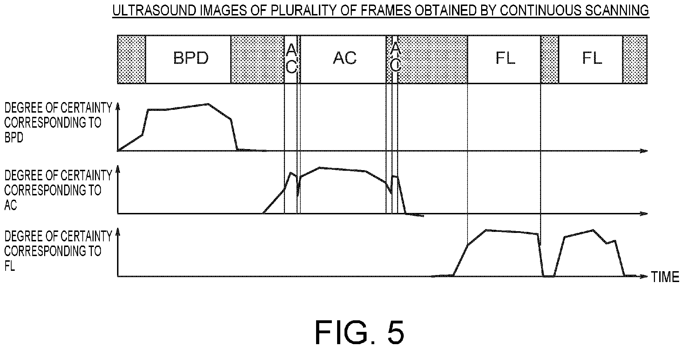

FIG. 5 is a diagram showing a step of calculating degrees of certainty in the operation example of the ultrasonic diagnostic apparatus according to the first embodiment. In the example shown in FIG. 5, the degree-of-certainty calculation function 532 calculates a degree of certainty corresponding to BPD, that is, a degree of certainty that a measurement cross section of BPD will be included in the ultrasound image. Further, the degree-of-certainty calculation function 532 calculates a degree of certainty corresponding to AC, that is, a degree of certainty that a measurement cross section of AC will be included in the ultrasound image. Further, the degree-of-certainty calculation function 532 calculates a degree of certainty corresponding to FL, that is, a degree of certainty that a measurement cross section of FL will be included in the ultrasound image.

After degrees of certainty are calculated, as shown in FIG. 2, the division function 533 performs, based on the calculated degrees of certainty, blocking that divides ultrasound images into blocks (step S3).

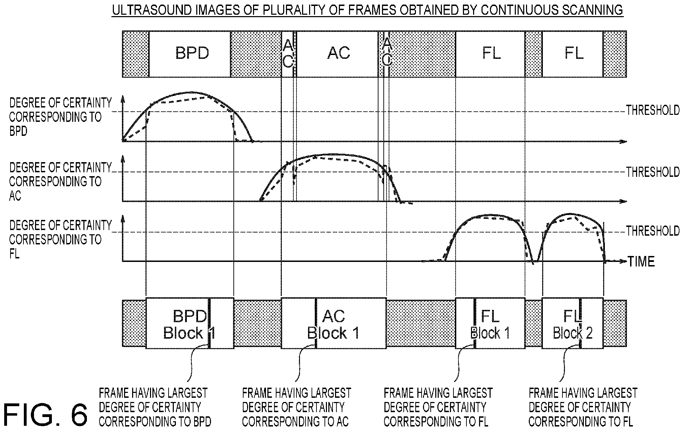

FIG. 6 is a diagram showing a step of performing blocking on ultrasound images in the operation example of the ultrasonic diagnostic apparatus 1 according to the first embodiment. In the example shown in FIG. 6, the division function 533 interpolates the degrees of certainty calculated by the degree-of-certainty calculation function 532 with an nth-degree polynomial, and thereby approximates the degrees of certainty to a smooth spline curve. By subjecting the degrees of certainty to curve approximation, slightly discrete frames of the same examination object can be combined into one block (that is, an ultrasound image group composed of two or more frames including a common examination object). After degrees of certainty are subjected to curve approximation, the division function 533 compares the degree of certainty subjected to curve approximation with a threshold of the degree of certainty. Then, the division function 533 classifies ultrasound images falling under a group of frames in which the degree of certainty is equal to or more than a threshold into the same block, and thereby performs blocking. Specifically, the division function 533 classifies ultrasound images falling under a group of frames in which the degree of certainty corresponding to BPD is equal to or more than a threshold into a block of BPD, "BPD Block 1". Further, the division function 533 classifies ultrasound images falling under a group of frames in which the degree of certainty corresponding to AC is equal to or more than a threshold into a block of AC, "AC Block 1". Further, the division function 533 classifies ultrasound images falling under a group of frames in which the degree of certainty corresponding to FL is equal to or more than a threshold into a block of FL, "FL Block 1". Further, the division function 533 classifies ultrasound images falling under another group of frames in which the degree of certainty corresponding to FL is equal to or more than the threshold into another block of FL, "FL Block 2".

After blocking is performed, as shown in FIG. 2, the display control function 534 displays block images (step S4). FIG. 7 is a diagram showing a step of displaying block images in the operation example of the ultrasonic diagnostic apparatus 1 according to the first embodiment. In the example shown in FIG. 7, in a screen A including an ultrasound image B, the display control function 534 displays a schematic image C schematically showing blocks. In the example shown in FIG. 7, the ultrasound image B is an image corresponding to FL. The schematic image C of blocks includes a band-shaped image C1 representing a time axis, rectangular block images C2 representing block main bodies superimposed on the image C1, and frames C3 having the largest degrees of certainty on the block images C2. In other words, the display control function 534 displays examination objects (BPD, AC, FL, etc.) shown by ultrasound image groups and the positions (blocks) on the time axis of the ultrasound image groups in a distinguishable manner. Further, the display control function 534 displays the positions on the time axis of ultrasound images for performing examination (frames C3) in a distinguishable manner. Note that the displaying of the images C1 and C3, which are other than the block images C2, may be omitted. In the example shown in FIG. 7, the display control function 534 further displays a frame number "#511" of the last frame in the schematic image C of blocks. Further, in the example shown in FIG. 7, the display control function 534 further displays a mark D indicating a frame corresponding to the ultrasound image B in the schematic image C of blocks. Further, in the example shown in FIG. 7, the display control function 534 further displays a measurement caliper E on a region where automatic detection (that is, automatic measurement) of the examination object by the degree-of-appropriateness calculation function 535 has been performed.

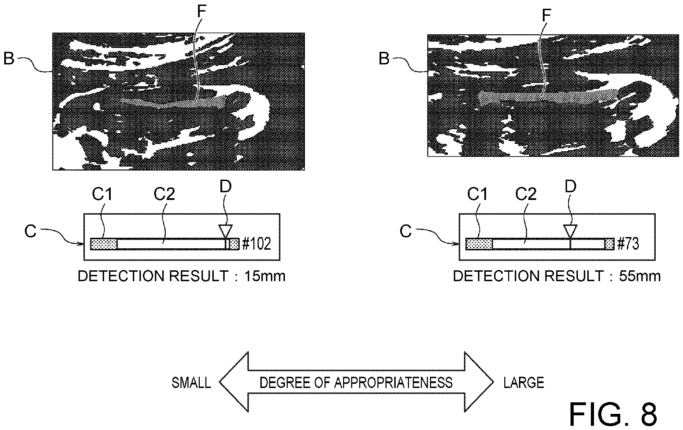

After block images are displayed, as shown in FIG. 2, the degree-of-appropriateness calculation function 535 calculates the degree of appropriateness of each frame for each block (step S5). FIG. 8 is a diagram showing a step of calculating degrees of appropriateness in the operation example of the ultrasonic diagnostic apparatus 1 according to the first embodiment. In the example shown in FIG. 8, the degree-of-appropriateness calculation function 535 performs automatic detection (that is, automatic measurement) of the examination object on cross sections F corresponding to the examination object in the ultrasound image B. Then, based on the detection result of automatic detection, the degree-of-appropriateness calculation function 535 calculates the degree of appropriateness of each frame. For example, the degree-of-appropriateness calculation function 535 performs calculation such that the degree of appropriateness of a frame in which the result of FL detection is 55 mm is a value larger than the degree of appropriateness of a frame in which the result of FL detection is 15 mm.

After degrees of appropriateness are calculated, as shown in FIG. 2, the determination function 536 determines, based on the calculated degrees of appropriateness, ultrasound images for performing examination of examination objects (step S6). For example, the determination function 536 determines that the ultrasound image of a frame having the largest degree of appropriateness in a block is an ultrasound image for performing examination of the examination object.

After ultrasound images for performing examination of examination objects are determined, the display control function 534 determines whether a freeze operation by the operator is performed or not (step S7).

In the case where a freeze operation is performed (step S7: YES), the display control function 534 displays an ultrasound image for performing examination of the examination object of one block (step S8). The one block is, for example, the oldest or newest block on a time series basis. On the other hand, in the case where a freeze operation is not performed (step S7: NO), the image generation function 531 generates and displays a new ultrasound image (step S1).

After displaying an ultrasound image for performing examination of the examination object of one block, the display control function 534 determines whether a confirmation operation by the operator is performed or not (step S9). The confirmation operation is an operation of confirming that the displayed ultrasound image for performing examination of the examination object is to be used for examination.

In the case where a confirmation operation is performed (step S9: YES), the display control function 534 determines whether or not there is a next block in which an ultrasound image for performing examination of the examination object has yet to be displayed (step S10). On the other hand, in the case where a confirmation operation is not performed (step S9: NO), the display control function 534 repeats the determination as to whether a confirmation operation is performed or not (step S9).

In the case where there is a next block (step S10: YES), the display control function 534 displays an ultrasound image for performing examination of the examination object of the next block (step S8). On the other hand, in the case where there is no next block (step S10: NO), the display control function 534 ends the processing.

As described hereinabove, in the first embodiment, for each of ultrasound images of a plurality of frames acquired by continuous scanning of a subject P using the ultrasonic probe 2, the degree-of-appropriateness calculation function 535 calculates a degree of appropriateness indicating how appropriate the examination for the examination object is. Based on the degrees of appropriateness calculated by the degree-of-appropriateness calculation function 535, the determination function 536 determines ultrasound images for performing examination of examination objects. More specifically, the determination function 536 determines ultrasound images for performing examination of examination objects from among the ultrasound images of a plurality of frames.

Thus, the operator does not need to repeat the operation of the ultrasonic probe 2 on the subject P and the freeze operation of the apparatus body 5 for every examination object. Therefore, examination of examination objects based on ultrasound images can be simply and quickly performed.

In the first embodiment, the division function 533 divides ultrasound images of a plurality of frames into blocks each showing a common attribute. The degree-of-appropriateness calculation function 535 calculates degrees of appropriateness for each block divided by the division function 533. Based on the degrees of appropriateness calculated by the degree-of-appropriateness calculation function 535, the determination function 536 determines an ultrasound image for performing examination of the examination object for each block divided (that is, distinguished) by the division function 533.

Thereby, for each block, degrees of appropriateness can be efficiently calculated, and an ultrasound image for performing examination of the examination object can be efficiently determined. Therefore, examination of examination objects based on ultrasound images can be more simply and quickly performed.

In the first embodiment, the common attribute shown by the block is a common examination object. The degree-of-certainty calculation function 532 calculates a degree of certainty that a cross section corresponding to the examination object will be included in the ultrasound image. Based on the degrees of certainty calculated by the degree-of-certainty calculation function 532, the division function 533 divides ultrasound images of a plurality of frames into blocks each showing a common examination object.

Thus, based on the degrees of certainty, ultrasound images of a plurality of frames can be divided (that is, classified) into appropriate blocks each showing a common examination object. Therefore, examination of examination objects based on ultrasound images can be more appropriately performed.

In the first embodiment, the display control function 534 displays block images showing blocks divided by the division function 533.

Thereby, the locations of cross sections corresponding to an examination object among the ultrasound images of a plurality of frames can be visualized, and thus convenience can be improved.

In the first embodiment, the degree-of-appropriateness calculation function 535 may calculate the degree of appropriateness based on a detection value (that is, a measurement value) of the examination object.

Thereby, the degree of appropriateness can be appropriately calculated.

In the first embodiment, the block image is an image schematically showing a block. The display control function 534 arranges and displays a plurality of block images according to a time series.

Thereby, the operator can easily grasp the locations of cross sections corresponding to an examination object, and thus convenience can be further improved. Note that, together with the block images, the display control function 534 may display, on each block image, a frame C3 of which the degree of certainty calculated by the degree-of-certainty calculation function 534 is largest. Thereby, the operator can easily grasp the locations of ultrasound images for performing examination of examination objects, and thus convenience can be further improved.

In the first embodiment, the display control function 534 may vary the display color of the block image according to the examination object.

Thereby, for each type of examination object, the locations of cross sections for the examination object can be visualized in an easy-to-understand manner for the operator; thus, convenience can be further improved.

In the first embodiment, according to an operation of the operator, the display control function 534 displays an ultrasound image for performing examination of an examination object determined by the determination function 536.

Thereby, the ultrasound image for performing examination of the examination object can be visualized, and thus convenience can be improved.

A plurality of modification examples described below can be applied to the ultrasonic diagnostic apparatus 1 according to the first embodiment.

### (First modification example of the first embodiment)

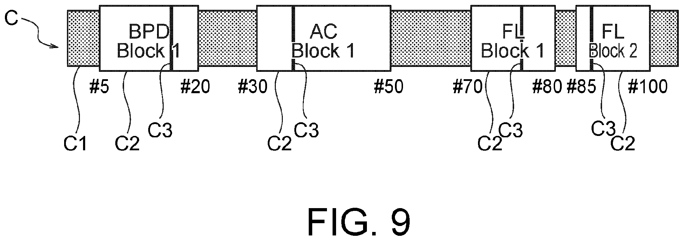

First, a first modification example of the first embodiment in which a block image showing a head frame number and a tail frame number in a block is displayed is described focusing on a difference from the above-described embodiment. FIG. 9 is a diagram showing a step of displaying block images in an operation example of an ultrasonic diagnostic apparatus 1 according to the first modification example of the first embodiment.

In the example shown in FIG. 9, the display control function 534 displays, as a plurality of block images C2, images each including a head frame number and a tail frame number in the block. Specifically, the display control function 534 displays a head frame number "#5" and a tail frame number "#20" of a block "BPD Block 1" while associating them with a block image C2 of the block "BPD Block 1". Further, the display control function 534 displays a head frame number "#30" and a tail frame number "#50" of a block "AC Block 1" while associating them with a block image C2 of the block "AC Block 1". Further, the display control function 534 displays a head frame number "#70" and a tail frame number "#80" of a block "FL Block 1" while associating them with a block image C2 of the block "FL Block 1". Further, the display control function 534 displays a head frame number "#85" and a tail frame number "#100" of a block "FL Block 2" while associating them with a block image C2 of the block "FL Block 2".

As described hereinabove, in the first modification example of the first embodiment, the block image is an image showing a head frame number and a tail frame number in the block.

Thereby, the frame number of a cross section corresponding to an examination object can be visualized, and thus convenience can be further improved.

### (Second modification example of the first embodiment)

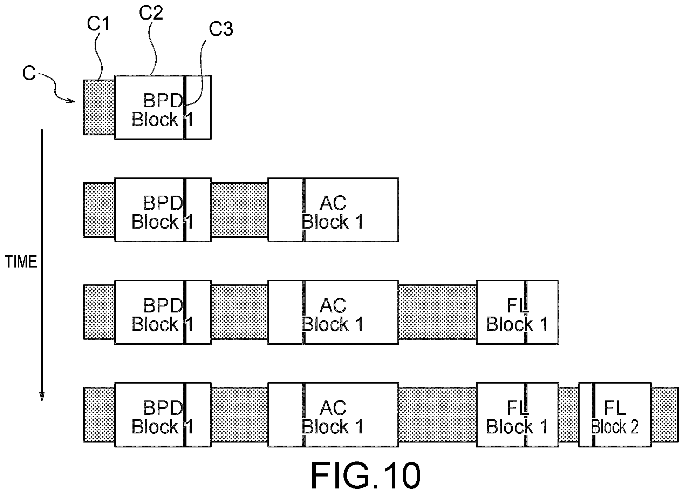

Next, a second modification example of the first embodiment in which blocking is performed in real time is described focusing on a difference from the above-described embodiment. FIG. 10 is a diagram showing a step of displaying block images in an operation example of an ultrasonic diagnostic apparatus 1 according to the second modification example of the first embodiment.

In the above-described embodiment, an example is described in which ultrasound images are subjected to blocking after ultrasound images of a predetermined number of frames in series are generated and displayed. In contrast, in the second modification example of the first embodiment, the division function 533 divides ultrasound images of a plurality of frames into blocks in real time during scanning. The display control function 534 displays block images together with ultrasound images in real time during scanning. In the example shown in FIG. 10, when the classification of ultrasound images into blocks is updated according to the progress of scanning (that is, the lapse of time), the display control function 534 adds and displays a block image C2 of the block of the update each time. Note that, also calculation of the degree of appropriateness by the degree-of-appropriateness calculation function 535 and determination of an ultrasound image for performing examination of the examination object by the determination function 536 may be performed in real time.

As described hereinabove, in the second modification example of the first embodiment, the division function 533 divides ultrasound images of a plurality of frames into blocks in real time during scanning. Further, the display control function 534 displays block images together with ultrasound images in real time during scanning.

Thereby, the change of the examination object according to the progress of scanning can be visualized, and thus convenience can be improved.

### (Third modification example of the first embodiment)

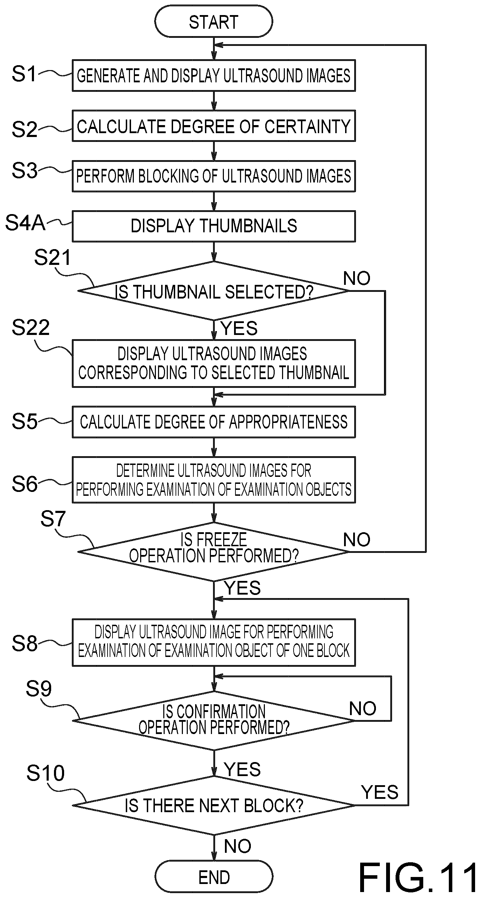

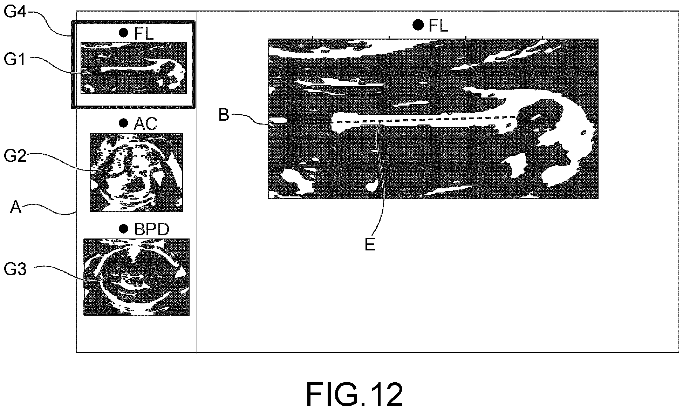

Next, a third modification example of the first embodiment in which a thumbnail of an ultrasound image of each block is displayed is described focusing on a difference from the above-described embodiment. FIG. 11 is a flowchart showing an operation example of an ultrasonic diagnostic apparatus 1 according to the third modification example of the first embodiment. FIG. 12 is a diagram showing a step of displaying thumbnails in the operation example of the ultrasonic diagnostic apparatus 1 according to the third modification example of the first embodiment.

In the above-described embodiment, an example in which the block image is an image schematically showing a block is described. In contrast, in the third modification example of the first embodiment, the block image is a thumbnail of an ultrasound image of each block. The thumbnail is, for example, an image obtained by contracting the ultrasound image (that is, a still image) of the head frame of each block.

As shown in FIG. 11, after ultrasound images are subjected to blocking, the display control function 534 displays a thumbnail corresponding to each block such that the thumbnail is selectable by an operation of the operator (step S4A). After displaying thumbnails, the display control function 534 determines whether a thumbnail is selected by an operation of the operator or not (step S21).

In the case where a thumbnail is selected (step S21: YES), the display control function 534 displays ultrasound images corresponding to the selected thumbnail (step S22). That is, the display control function 534 reproduces a plurality of ultrasound images belonging to the selected thumbnail, and displays them in a moving image form. On the other hand, in the case where a thumbnail is not selected (step S21: NO), the degree-of-appropriateness calculation function 535 calculates the degree of appropriateness (step S5).

In the example shown in FIG. 12, the display control function 534 displays thumbnail G1 corresponding to FL, thumbnail G2 corresponding to AC, and thumbnail G3 corresponding to BPD in a selectable manner. When, for example, selection of thumbnail G1 is accepted by the input interface 3, the display control function 534 reproduces ultrasound images B belonging to the selected thumbnail G1. The display control function 534 may display an image G4 indicating that thumbnail G1 is selected, while associating image G4 with the selected thumbnail G1.

As described hereinabove, in the third modification example of the first embodiment, the block image is a thumbnail of an ultrasound image of each block. The display control function 534 displays thumbnails such that a thumbnail is selectable by an operation of the operator, and displays ultrasound images corresponding to a thumbnail selected by an operation of the operator.

Thereby, the locations of cross sections corresponding to an examination object among the ultrasound images of a plurality of frames can be visualized. Further, an ultrasound image having a cross section corresponding to a desired examination object can be selected and displayed. Thus, convenience can be further improved.

### (Fourth modification example of the first embodiment)

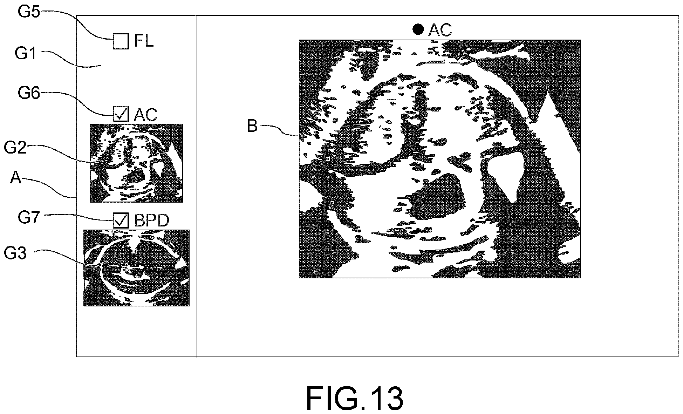

Next, a fourth modification example of the first embodiment in which a detected examination object is visualized is described focusing on a difference from the above-described embodiment. FIG. 13 is a diagram showing check boxes of detected examination objects in an operation example of an ultrasonic diagnostic apparatus 1 according to the fourth modification example of the first embodiment.

In the example shown in FIG. 13, the display control function 534 displays, on thumbnail G1 corresponding to FL, check box G5 indicating whether automatic detection (that is, automatic measurement) of FL is performed or not. Further, the display control function 534 displays, on thumbnail G2 corresponding to AC, check box G6 indicating whether automatic detection of AC is performed or not. Further, the display control function 534 displays, on thumbnail G3 corresponding to BPD, check box G7 indicating whether automatic detection of BPD is performed or not.

In the example shown in FIG. 13, the operator can, based on check boxes G5, G6, and G7, easily grasp an examination object for which automatic detection has been performed (AC and BPD in FIG. 13) and an examination object for which automatic detection has yet to be performed (FL in FIG. 13). Thus, convenience can be further improved.

### (Fifth modification example of the first embodiment)

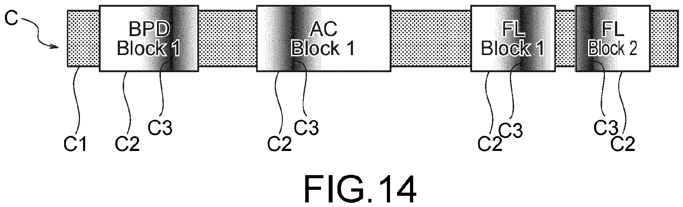

Next, a fifth modification example of the first embodiment in which the display color of the block image is varied according to the degree of certainty is described focusing on a difference from the above-described embodiment. FIG. 14 is a diagram showing a step of displaying block images in an operation example of an ultrasonic diagnostic apparatus 1 according to the fifth modification example of the first embodiment.

In the fifth modification example of the first embodiment, the display control function 534 varies the display color of the block image according to the degree of certainty calculated by the degree-of-certainty calculation function 532. In the example shown in FIG. 14, the display control function 534 displays the block image C2 in gradation according to the degree of certainty calculated by the degree-of-certainty calculation function 532. For example, the display control function 534 displays the block image C2 in gradation such that the display color becomes gradually darker with proximity to a frame having the largest degree of certainty (that is, image C3).

As described hereinabove, in the fifth modification example of the first embodiment, the display control function 534 varies the display color of the block image according to the degree of certainty calculated by the degree-of-certainty calculation function 532. Thereby, the degree of certainty can be visualized, and thus convenience can be further improved. Further, the display control function 534 displays the block image in gradation according to the degree of certainty calculated by the degree-of-certainty calculation function 532. Thereby, changes in the degree of certainty in the block can be visualized, and thus convenience can be further improved.

### (Sixth modification example of the first embodiment)

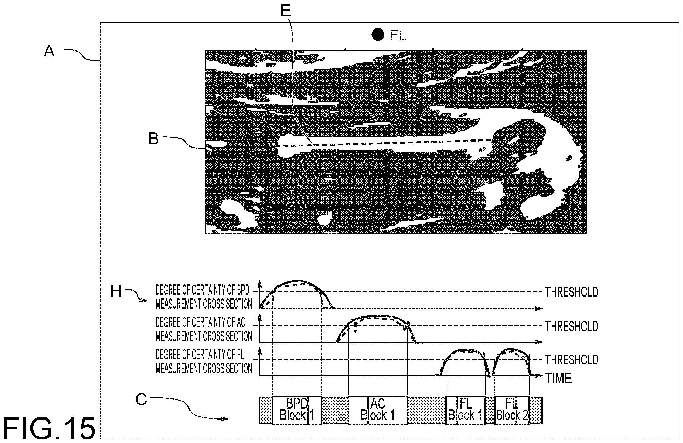

Next, a sixth modification example of the first embodiment in which a graph of degrees of certainty is displayed is described focusing on a difference from the above-described embodiment. FIG. 15 is a diagram showing a step of displaying a graph showing degrees of certainty in an operation example of an ultrasonic diagnostic apparatus 1 according to the sixth modification example of the first embodiment.

In the sixth modification example of the first embodiment, the display control function 534 further displays, in addition to block images, a graph showing degrees of certainty calculated by the degree-of-certainty calculation function 532 while associating the graph with the block images. In the example shown in FIG. 15, the display control function 534 displays a graph H showing changes in the degree of certainty in blocks while associating the graph H with the schematic image C of blocks.

As described hereinabove, in the sixth modification example of the first embodiment, the display control function 534 displays a graph showing degrees of certainty calculated by the degree-of-certainty calculation function 532.

Thereby, the operator can easily grasp changes in the degree of certainty in blocks, and thus convenience can be further improved.

### (Seventh modification example of the first embodiment)

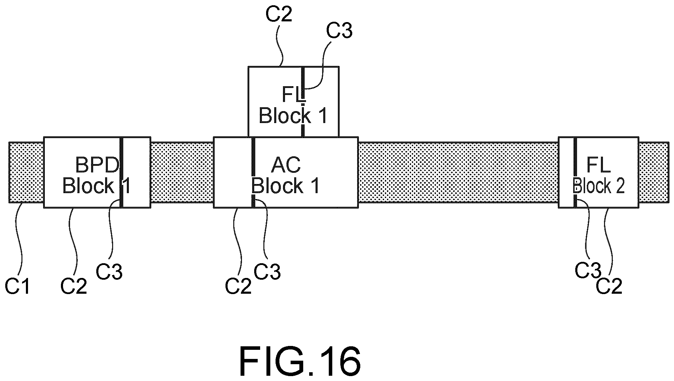

Next, a seventh modification example of the first embodiment in which a plurality of examination objects simultaneously included in ultrasound images are individually subjected to blocking is described focusing on a difference from the above-described embodiment. FIG. 16 is a diagram showing a step of displaying block images in an operation example of an ultrasonic diagnostic apparatus 1 according to the seventh modification example of the first embodiment.

In the seventh modification example of the first embodiment, in the case where a plurality of examination objects are simultaneously included in ultrasound images of a plurality of frames, the division function 533 divides the ultrasound images of a plurality of frames into blocks individually for the plurality of examination objects. Then, the display control function 534 displays block images of the plurality of examination objects in an overlapping manner or in a side-by-side manner.

In the example shown in FIG. 16, FL and AC are simultaneously included in ultrasound images of a plurality of frames. That is, over a plurality of frames in series, a cross section corresponding to FL and a cross section corresponding to AC are included in the ultrasound image of the same frame. Since FL and AC are simultaneously included in ultrasound images of a plurality of frames, the division function 533 performs blocking on the ultrasound images of a plurality of frames individually for FL and AC. The display control function 534 displays a block image C2 of a block "FL Block 1" corresponding to FL and a block image C2 of a block "AC Block 1" corresponding to AC side by side.

As described hereinabove, in the seventh modification example of the first embodiment, in the case where a plurality of examination objects are simultaneously included in ultrasound images of a plurality of frames, the division function 533 divides the ultrasound images of a plurality of frames into blocks individually for the plurality of examination objects. The display control function 534 displays block images of the plurality of examination objects in an overlapping manner or in a side-by-side manner.

Thereby, also in the case where a plurality of examination objects are simultaneously included, the locations of cross sections corresponding to an examination object among the ultrasound images of a plurality of frames can be visualized, and thus convenience can be further improved.

### (Eighth modification example of the first embodiment)

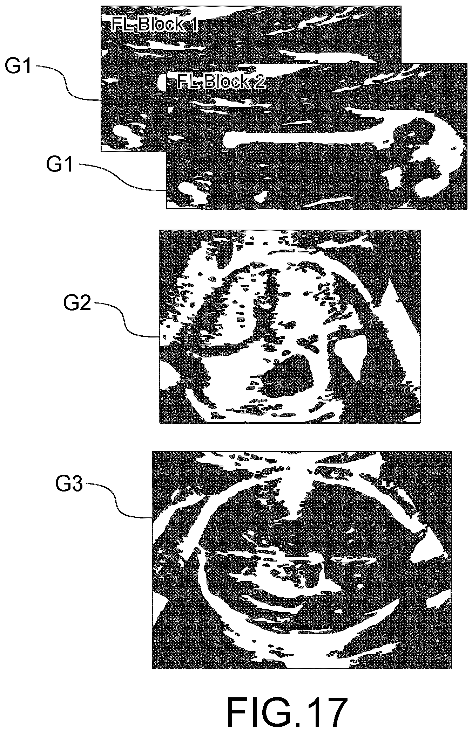

FIG. 17 is a diagram showing a step of displaying thumbnails in an operation example of an ultrasonic diagnostic apparatus 1 according to an eighth modification example of the first embodiment. As shown in FIG. 17, the display control function 534 may display a thumbnail G1 of a block "FL Block 1" corresponding to FL and a thumbnail G1 of a block "FL Block 2" corresponding to FL, which are simultaneously included in ultrasound images of a plurality of frames, in an overlapping manner. Note that in FIG. 17, the display control function 534 may compare the degrees of appropriateness between the most appropriate cross sections of the blocks simultaneously included in ultrasound images of a plurality of frames. Alternatively, the display control function 534 may compare the average values of the degrees of appropriateness of all the frames of the blocks simultaneously included in ultrasound images of a plurality of frames. Then, based on the result of comparison, the display control function 534 may change the order of displaying of the thumbnails of the blocks. For example, the display control function 534 may display the thumbnail of the block having the highest degree of appropriateness of the most appropriate cross section or the thumbnail of the block having the highest average value of the degree of appropriateness on the frontmost side.

Also in the example shown in FIG. 17, the locations of cross sections corresponding to an examination object among the ultrasound images of a plurality of frames can be visualized like in FIG. 16, and thus convenience can be further improved.

### (Ninth modification example of the first embodiment)

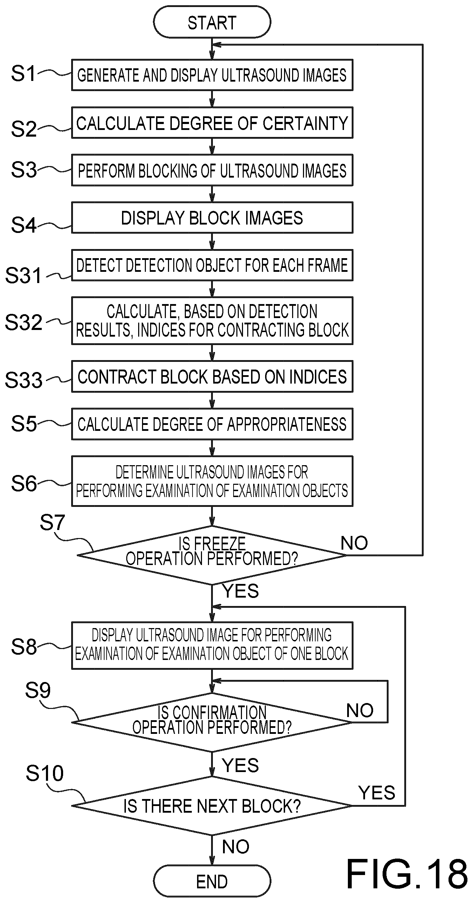

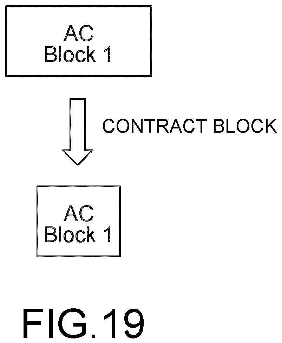

Next, a ninth modification example of the first embodiment in which the block is contracted based on detection values of the examination object is described focusing on a difference from the above-described embodiment. FIG. 18 is a flowchart showing an operation example of an ultrasonic diagnostic apparatus according to the ninth modification example of the first embodiment. FIG. 19 is a diagram showing a step of contracting a block in the operation example of the ultrasonic diagnostic apparatus 1 according to the ninth modification example of the first embodiment.

In the ninth modification example of the first embodiment, the division function 533 contracts the block based on detection values (that is, measurement values) of the examination object included in the block. Then, the degree-of-appropriateness calculation function 535 calculates degrees of appropriateness for the block contracted by the division function 533.

Specifically, as shown in FIG. 18, the division function 533 automatically detects (that is, automatically measures) the examination object for each block (step S31).

After performing automatic detection, based on the detection results, the division function 533 calculates indices for contracting the block (step S32). For example, the division function 533 may calculate, as an index, the difference of the detection result with respect to a reference value of the examination object acquired in advance (for example, the average value of the examination object of fetuses at a certain number of pregnancy weeks). Alternatively, the division function 533 may calculate, as an index, variance or standard deviation in the block of the detection result of each frame.

After calculating indices, the division function 533 contracts the block based on the calculated indices (step S33). For example, the division function 533 contracts the block such that only frames of which the index is equal to or less than a threshold are included in the block. In the example shown in FIG. 19, the division function 533 contracts a block "AC Block 1" corresponding to AC such that only frames of which the index is equal to or less than a threshold are included in the block "AC Block 1".

After the block is contracted, as shown in FIG. 18, the degree-of-appropriateness calculation function 535 calculates degrees of appropriateness for the contracted block (step S5).

As described hereinabove, in the ninth modification example of the first embodiment, the division function 533 contracts the block based on measurement values of the examination object included in the block. Further, the degree-of-appropriateness calculation function 535 calculates degrees of appropriateness for the block contracted by the division function 533.

Thereby, degrees of appropriateness can be appropriately calculated based on the block contracted based on the measurement result of the examination object, and thus an ultrasound image for performing examination of the examination object can be appropriately determined. Thereby, examination of examination objects based on ultrasound images can be more appropriately performed.

### (Second embodiment)

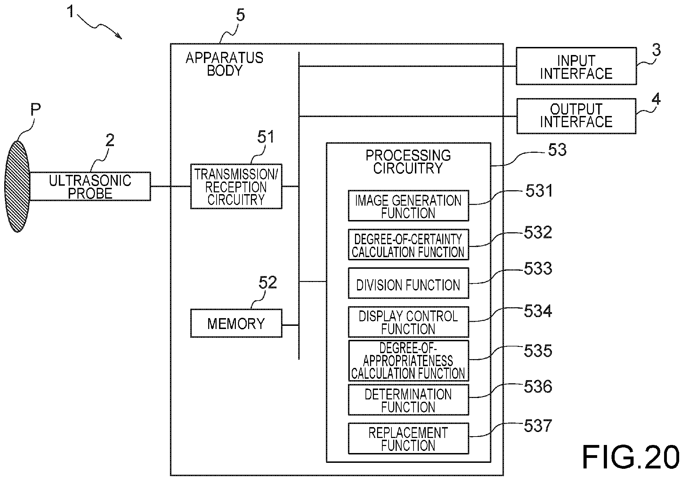

Next, a second embodiment in which an ultrasound image of an appropriate cross section is replaced according to an operation of the operator is described focusing on a difference from the above-described embodiment. FIG. 20 is a block diagram showing a configuration example of an ultrasonic diagnostic apparatus 1 according to the second embodiment.

As shown in FIG. 20, a processing circuitry 53 of the ultrasonic diagnostic apparatus 1 according to the second embodiment further includes a replacement function 537 in addition to the configuration of the first embodiment. The replacement function 537 is an example of a replacement part.

The replacement function 537 replaces, as an ultrasound image for performing examination of the examination object, the ultrasound image of a frame selected by the operator from a block image displayed by the display control function 534 with an ultrasound image for performing examination of the examination object determined by the determination function 536.

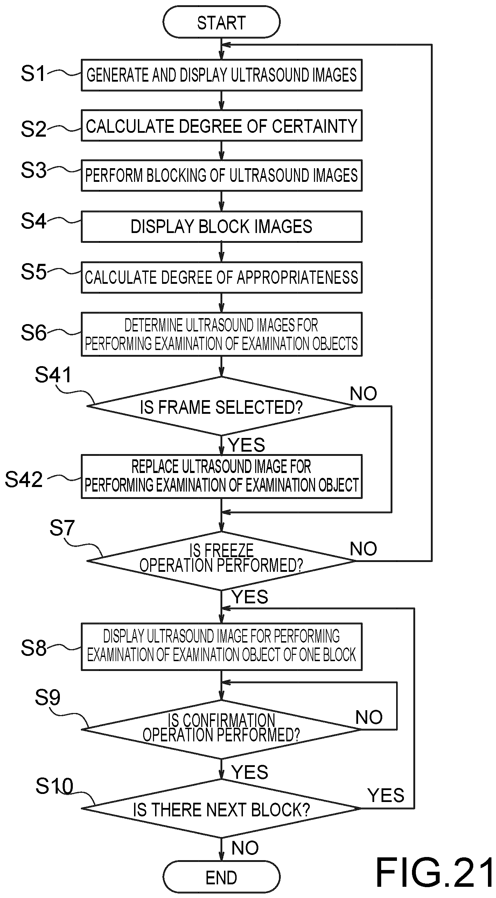

FIG. 21 is a flowchart showing an operation example of the ultrasonic diagnostic apparatus 1 according to the second embodiment. Specifically, in the example shown in FIG. 21, after ultrasound images for performing examination of examination objects are determined by the determination function 536, the replacement function 537 determines whether a frame in a block image is selected by the operator or not (step S41).

In the case where a frame is selected (step S41: YES), the replacement function 537 replaces, as an ultrasound image for performing examination of the examination object, the ultrasound image of the selected frame with the ultrasound image determined by the determination function 536 (step S42). On the other hand, in the case where no frame is selected (step S41: NO), the display control function 534 determines whether a freeze operation is performed or not (step S7).

As described hereinabove, in the second embodiment, the replacement function 537 replaces, as an ultrasound image for performing examination of the examination object, the ultrasound image of a frame selected by the operator from a block image displayed by the display control function 534 with an ultrasound image for performing examination of the examination object determined by the determination function 536.

Thereby, an ultrasound image according to a desire of the operator can be used for examination of the examination object, and thus the flexibility of examination of examination objects can be improved.

### (Third embodiment)

Next, a third embodiment in which, based on time, ultrasound images of a plurality of frames are divided into blocks each showing a common time phase is described focusing on a difference from the above-described embodiment. In the above-described embodiment, an example is described in which ultrasound images of a plurality of frames are divided into blocks each showing a common examination object as an example of the common attribute. In contrast, a division function 533 in the third embodiment divides ultrasound images of a plurality of frames into blocks each showing a common time phase as another example of the common attribute.

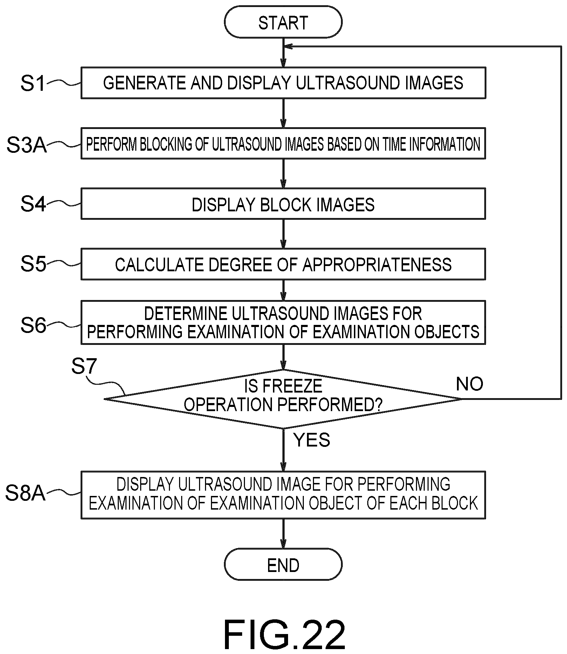

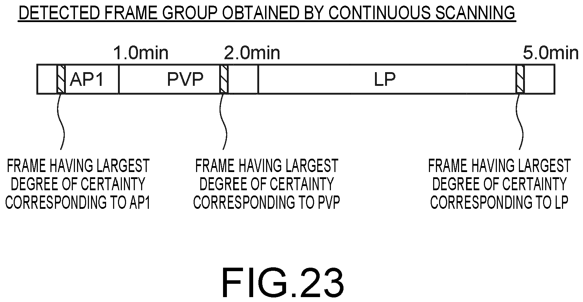

FIG. 22 is a flowchart showing an operation example of an ultrasonic diagnostic apparatus 1 according to the third embodiment. Specifically, as shown in FIG. 22, the division function 533 performs blocking on ultrasound images based on time information (step S3A). FIG. 23 is a diagram showing a step of performing blocking on ultrasound images in the operation example of the ultrasonic diagnostic apparatus 1 according to the third embodiment. In the example shown in FIG. 23, the division function 533 divides ultrasound images of a plurality of frames generated by continuous scanning of the subject in a contrast-enhanced ultrasonic examination into blocks each showing a common time phase (an arterial phase, AP; a portal venous phase, PVP; or a late phase, LP). Further, in the example shown in FIG. 23, for each of the blocks divided for time phases based on time information, a frame having the largest degree of certainty of the examination object, such as a lesion portion, calculated by the degree-of-certainty calculation function 532 is specified.

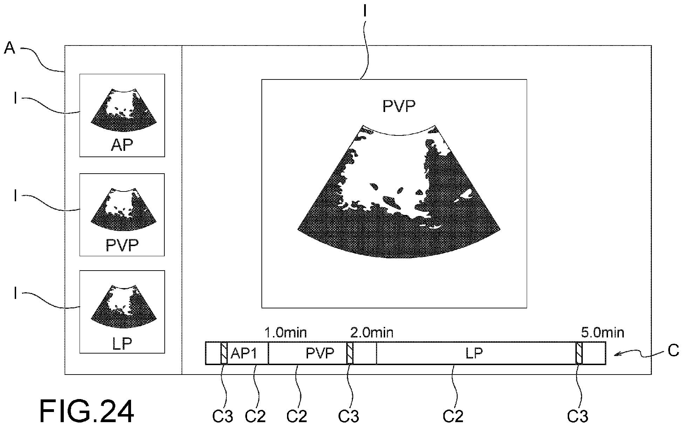

As shown in FIG. 22, in the case where a freeze operation is performed (step S7: YES), the display control function 534 displays an ultrasound image for performing examination of the examination object of each block determined by the determination function 536 (step S8A). Note that the ultrasound image for performing examination of the examination object may be determined based on, for example, the above-described degree of certainty calculated by the degree-of-certainty calculation function 532. FIG. 24 is a diagram showing a step of displaying ultrasound images for performing examination of examination objects in the operation example of the ultrasonic diagnostic apparatus 1 according to the third embodiment. In the example shown in FIG. 24, the display control function 534 displays ultrasound images I for performing examination of examination objects of time phases. Further, in the example shown in FIG. 24, the display control function 534 displays, as a schematic image C schematically showing blocks, an image including block images C2 for pieces of time information and a frame C3 having the largest degree of certainty on each block image C2.

As described hereinabove, in the third embodiment, based on time, the division function 533 divides ultrasound images of a plurality of frames into blocks each showing a common time phase. Thereby, the flexibility of examination of an examination object using ultrasound images for performing examination of the examination object can be improved.

Note that the term "processor" used in the above description means, for example, a CPU (central processing unit), a GPU (graphics processing unit), or a circuitry such as an application-specific integrated circuit (ASIC) or a programmable logic device (for example, a simple programmable logic device (SPLD), a complex programmable logic device (CPLD), or a field-programmable gate array (FPGA)). The processor implements a function by reading and executing a program stored in a storage circuitry. Note that, instead of storing a program in a storage circuitry, a program may be directly incorporated in the circuitry of the processor. In this case, the processor implements a function by reading and executing a program incorporated in the circuitry. Note that the processor is not limited to a case of being configured as a single processor circuitry, and a plurality of independent circuitries may be combined and configured as one processor to implement the functions. Further, the plurality of constituent elements in FIG. 1 may be integrated into one processor to implement the functions.

According to at least one of the embodiments described above, examination of examination objects based on ultrasound images can be simply and quickly performed.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the scope of the inventions as defined by the appended claims.

## Claims

1. An ultrasonic diagnostic apparatus comprising a processing circuitry configured to:
acquire ultrasound images of a plurality of frames by continuous scanning of a subject using an ultrasonic probe; and
distinguishably display positions on a time axis, where ultrasound image groups have been acquired, for each ultrasound image group which comprises two or more frames which include a common examination object among the plurality of frames.

2. The ultrasonic diagnostic apparatus according to claim 1, wherein
the processing circuitry is further configured to:
distinguish the examination object for each of the plurality of frames; and
distinguishably display the positions on the time axis where the ultrasound image groups have been acquired, for each ultrasound image group, based on a result of distinguishing the examination object.

3. The ultrasonic diagnostic apparatus according to claim 2, wherein
the processing circuitry is further configured to:
calculate, for each frame in which the examination object has been distinguished, a degree of appropriateness indicating how appropriate the examination is; and
determine, based on the calculated degrees of appropriateness, an ultrasound image for performing examination of the examination object.

4. The ultrasonic diagnostic apparatus according to claim 3, wherein
the processing circuitry is further configured to: distinguish the examination object by dividing the ultrasound images of the plurality of frames into blocks each showing a common attribute;
calculate the degree of appropriateness for each of the divided blocks; and
determine, for each of the divided blocks, based on the calculated degrees of appropriateness, an ultrasound image for performing examination of the examination object.

5. The ultrasonic diagnostic apparatus according to claim 4, wherein
the common attribute is a common examination object, and
the processing circuitry is further configured to: calculate a degree of certainty that a cross section corresponding to the examination object will be included in the ultrasound image; and
divide, based on the calculated degrees of certainty, the ultrasound images of the plurality of frames into the blocks each showing the common examination object.

6. The ultrasonic diagnostic apparatus according to claim 4, wherein
the common attribute is a common time phase, and
the processing circuitry is further configured to divide, based on time, the ultrasound images of the plurality of frames into the blocks each showing a common time phase.

7. The ultrasonic diagnostic apparatus according to claim 5, wherein the processing circuitry is configured to distinguishably display the positions on the time axis by displaying a block image showing the divided block.

8. The ultrasonic diagnostic apparatus according to claim 7, wherein the processing circuitry is further configured to vary a display color of the block image according to the calculated degree of certainty.

9. The ultrasonic diagnostic apparatus according to claim 8, wherein the processing circuitry is further configured to display the block image in gradation according to the calculated degree of certainty.

10. The ultrasonic diagnostic apparatus according to claim 7, wherein the processing circuitry is further configured to replace, as an ultrasound image for performing examination of the examination object, an ultrasound image of a frame selected by a user from the displayed block image with a determined ultrasound image for performing examination of the examination object.

11. The ultrasonic diagnostic apparatus according to claim 7, wherein
the processing circuitry is further configured to: divide the ultrasound images of the plurality of frames into the blocks in real time during the scanning; and
display the block image together with the ultrasound image in real time during the scanning.

12. The ultrasonic diagnostic apparatus according to claim 7, wherein
the processing circuitry is further configured to, in a case where a plurality of examination objects are simultaneously included in the ultrasound images of the plurality of frames, divide the ultrasound images of the plurality of frames into the blocks individually for the plurality of examination objects, and
display the block images of the plurality of examination objects in an overlapping manner or in a side-by-side manner.

13. The ultrasonic diagnostic apparatus according to claim 7, wherein the processing circuitry is further configured to display a graph showing the calculated degree of certainty.

14. The ultrasonic diagnostic apparatus according to claim 4, wherein
the processing circuitry is further configured to: contract the block based on detection values of the examination object included in the block; and
calculate the degree of appropriateness for the contracted block.

15. The ultrasonic diagnostic apparatus according to claim 14, wherein the processing circuitry is further configured to calculate the degree of appropriateness based on a detection value of the examination object.
